# EUROPEAN PATENT SPECIFICATION

(11) **EP 4 525 749 B1**
(45) Date of publication and mention of the grant of the patent: **14.10.2026**
(21) Application number: 23730933.1
(22) Date of filing: 18.05.2023
(51) Int. Cl.: A61B 17/32

(54) **SURGICAL SYSTEM INCLUDING A CORDLESS SURGICAL INSTRUMENT, COMMUNICATION HUB, AND ONE OR MORE CONNECTED DEVICES**
CHIRURGISCHES SYSTEM MIT EINEM SCHNURLOSEN CHIRURGISCHEN INSTRUMENT, KOMMUNIKATIONSNABE UND EINER ODER MEHREREN VERBUNDENEN VORRICHTUNGEN
SYSTÈME CHIRURGICAL COMPRENANT UN INSTRUMENT CHIRURGICAL SANS FIL, CONCENTRATEUR DE COMMUNICATION ET UN OU PLUSIEURS DISPOSITIFS CONNECTÉS

(30) Priority: 18.05.2022 US 202263343231 P
(43) Date of publication of application: 26.03.2025
(73) Proprietor: Covidien LP, Mansfield, MA 02048 (US)
(72) Inventor: VAN TOL, David J., Boulder, Colorado 80301 (US); CRAIG, Jason L., Lafayette, Colorado 80026 (US); MALANG, Keith W., Longmont, Colorado 80301 (US); NUESKEN, Rebecca A., Lafayette, Colorado 80026 (US); FAGAN, James R., Boulder, Colorado 80301 (US); COWLEY, Matthew S., Boulder, Colorado 80301 (US); WILLETT, Stacy L., Lafayette, Colorado 80026 (US); LYONS, Michael B., Boulder, Colorado 80301 (US); BRANNAN, Edward L., Lafayette, Colorado 80026 (US); HORTON, Titus C., Lafayette, Colorado 80026 (US); BRADLEY, Kristen P., Lafayette, Colorado 80026 (US); BARRETT, Graham J., Lafayette, Colorado 80026 (US); BROWN, Christopher T., Lafayette, Colorado 80026 (US)
(74) Representative: Gray, James
(86) International application number: PCT/IB2023/055121
(87) International publication number: WO 2023/223254

(56) References cited:
- EP-A1- 3 508 145
- US-A1- 2009 057 369
- US-A1- 2019 008 543
- US-A1- 2019 223 850
- US-A1- 2020 405 301

## Description

### FIELD

This disclosure relates to surgical systems and, more particularly, to a surgical system including a cordless surgical instrument, e.g., a cordless ultrasonic surgical instrument, a communication hub, and one or more connected devices.

### BACKGROUND

Many surgical instruments incorporate hardware and/or software features that facilitate operation and control. Such surgical instruments are also typically configured to obtain and store data related to the instrument and/or components thereof such as, for example: identification data, use data, and/or performance data. However, such surgical instruments are limited in their ability to process and/or convey information to a user solely from the surgical instruments themselves. Examples of cited art include: US 2019008543; EP 3508145; US2019/223850; US 2009/057369; US2020/405301. Particularly, US 2019008543 A1 discloses a medical device configured to determine if the device does not function properly after reassembly, and may include one or more sets of instructions to be executed by a processor located in the medical device. The instructions may embody one or more methods to test the medical device for proper reassembly and component function.

### SUMMARY

As used herein, the term "distal" refers to the portion that is being described which is farther from an operator (whether a human clinician or a surgical robot), while the term "proximal" refers to the portion that is being described which is closer to the operator. Terms including "generally," "about," "substantially," and the like, as utilized herein, are meant to encompass variations, e.g., manufacturing tolerances, material tolerances, use and environmental tolerances, measurement variations, design variations, and/or other variations, up to and including plus or minus 10 percent. To the extent consistent, any of the aspects described herein may be used in conjunction with any or all of the other aspects described herein.

The surgical system of the invention is defined by independent claim 1. Further embodiments are defined by the dependent claims. Provided in accordance with aspects of this disclosure is a surgical system including a cordless surgical instrument and a communication hub. The cordless surgical instrument is configured to obtain information regarding preparation of the cordless surgical instrument for use. The communication hub is configured to wirelessly connect to the cordless surgical instrument to receive the information therefrom. The communication hub is configured to instruct, based at least on the information, a connected device to provide an output relating to the preparation of the cordless surgical instrument for use.

According to the invention, the information regarding preparation of the cordless surgical instrument for use includes information regarding assembly of the cordless surgical instrument. In such aspects, the information may include information indicating that first and second components of the cordless surgical instrument are engaged with one another.

According to the invention, the output includes display of a plurality of steps for assembling the cordless surgical instrument for use. Further, the display is configured to switch between different steps of the plurality of steps based on additional information wirelessly communicated from the cordless surgical instrument to the communication hub.

In still another aspect of this disclosure, the information regarding preparation of the cordless surgical instrument for use includes identifying information for a plurality of components of the cordless surgical instrument. In such aspects, the output may include display of an indication of whether the plurality of components of the cordless surgical instrument is approved for use.

In yet another aspect of this disclosure, the information regarding preparation of the cordless surgical instrument for use includes information regarding an instrument test of the cordless surgical instrument. In such aspects, the output may include display of an indication of whether the cordless surgical instrument passed or failed the instrument test. In a case where the cordless surgical instrument failed the instrument test, the output may further include at least one of an indication of a cause of the failed instrument test or a troubleshooting recommendation.

In still yet another aspect of this disclosure, the cordless surgical instrument includes a battery assembly and a generator configured to drive the cordless surgical instrument. The generator is powered by the battery assembly. The cordless surgical instrument may further include an ultrasonic transducer and an ultrasonic blade coupled to the ultrasonic transducer. The generator is configured to drive the ultrasonic transducer to produce mechanical vibration motion at the ultrasonic blade.

Another surgical system provided in accordance with this disclosure includes a cordless surgical instrument configured to obtain information regarding at least one replaceable component of the cordless surgical instrument, and a communication hub configured to wirelessly connect to the cordless surgical instrument to receive the information therefrom. The communication hub is configured to instruct, based at least on the information, a connected device to provide an output relating to replacement of the at least one replaceable component of the cordless surgical instrument.

In an aspect of this disclosure, the at least one replaceable component is a battery assembly. In such aspects, the cordless surgical instrument may include a generator configured to drive the cordless surgical instrument, wherein the generator is powered by the battery assembly.

In another aspect of this disclosure, the information includes a state of charge of the battery assembly and the communication hub is configured to instruct, based at least on the state of charge of the battery assembly being below a threshold value, the connected device to provide the output. Alternatively, the information includes an indication that a state of charge of the battery assembly is below a threshold value.

In still another aspect of this disclosure, the output includes display of at least one step for replacing the at least one replaceable component.

The details of one or more aspects of this disclosure are set forth in the accompanying drawings and the description below. Other features, objects, and advantages of the techniques described in this disclosure will be apparent from the description and drawings, and from the claims.

### BRIEF DESCRIPTION OF DRAWINGS

Various aspects and features of this disclosure are described hereinbelow with reference to the drawings wherein like numerals designate identical or corresponding elements in each of the several views.
FIG. 1 is a schematic illustration of a surgical system provided in accordance with this disclosure;
FIG. 2 is a perspective view of a cordless ultrasonic surgical instrument configured for use with the surgical system of FIG. 1;
FIG. 3 is a longitudinal, cross-sectional view of a proximal portion of the cordless ultrasonic surgical instrument of FIG. 2;
FIG. 4 is a schematic illustration of portions of the cordless ultrasonic surgical instrument of FIG. 2;
FIG. 5 is a block diagram of a portion of the surgical system of FIG. 1; and
FIGS. 6A-12 are screen shots of various display screens provided in accordance with this disclosure.

### DETAILED DESCRIPTION

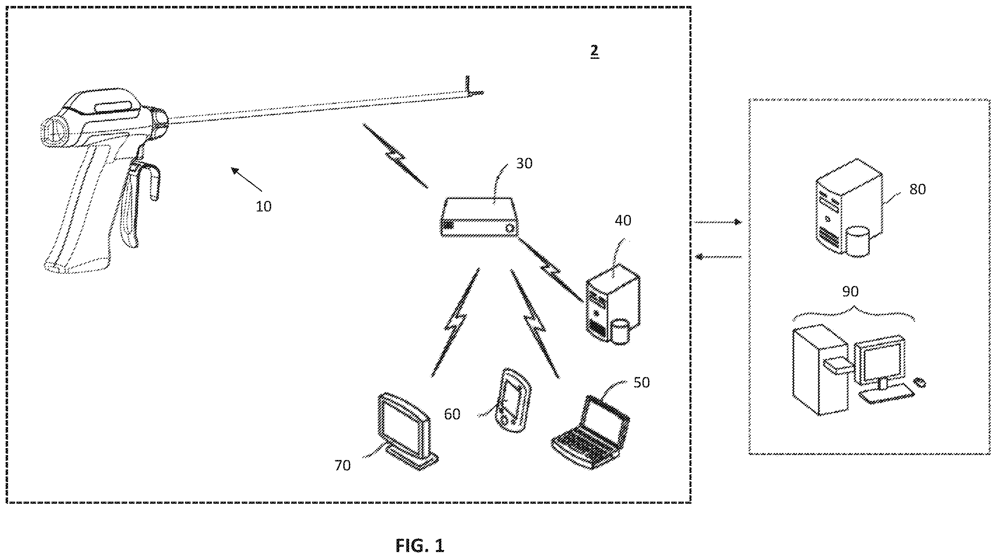

Turning to FIG. 1, a surgical system provided in accordance with the present disclosure is shown generally identified by reference numeral 2. Surgical system 2 includes one or more surgical instruments, e.g., a cordless ultrasonic surgical instrument 10; a communication hub 30; and one or more connected devices in communication with communication hub 30 such as, for example, a server 40, a computer 50, a smartphone or tablet 60, a display monitor 70, etc. Communication hub 30 and/or one or more of the connected devices 40-70 of surgical system 2 may further be configured to communicate with one or more remote and/or cloud based devices such as, for example, one or more servers 80 and/or one or more computers 90.

Cordless ultrasonic surgical instrument 10 is configured to wirelessly communicate with communication hub 30 using any suitable wireless protocol, e.g., radio frequency (RF), optical, Wi-Fi, Bluetooth^{®}, Bluetooth^{®} Low Energy, or ZigBee^{®}. Cordless ultrasonic surgical instrument 10 may be configured to communicate information to communication hub 30 such as, for example: identifying information of cordless ultrasonic surgical instrument 10 and/or components thereof; status information of cordless ultrasonic surgical instrument 10 and/or components thereof; use information associated with cordless ultrasonic surgical instrument 10 and/or components thereof; and/or other information associated with cordless ultrasonic surgical instrument 10 and/or components thereof.

Continuing with reference to FIG. 1, communication hub 30 communicates with each of the one or more connected devices 40-70 via a wired or wireless connection. With respect to wired connections, any suitable wired communication protocol may be utilized including, for example, transmission control protocol/internet protocol (TCP/IP), datagram protocol/internet protocol (UDP/IP), and/or datagram congestion control protocol (DCCP). With respect to wireless connections, any suitable wireless communication protocol may be utilized such as those detailed above.

Communication hub 30 may be configured to process information received from cordless ultrasonic surgical instrument 10 and to output the processed information to one or more of the connected devices 40-70 for display, further processing, control, notification, and/or any other suitable purposes. Although described with respect to cordless ultrasonic surgical instrument 10, the aspects and features of this disclosure are also applicable, to the extent consistent, for use with any other suitable surgical instrument communicating with communication hub 30.

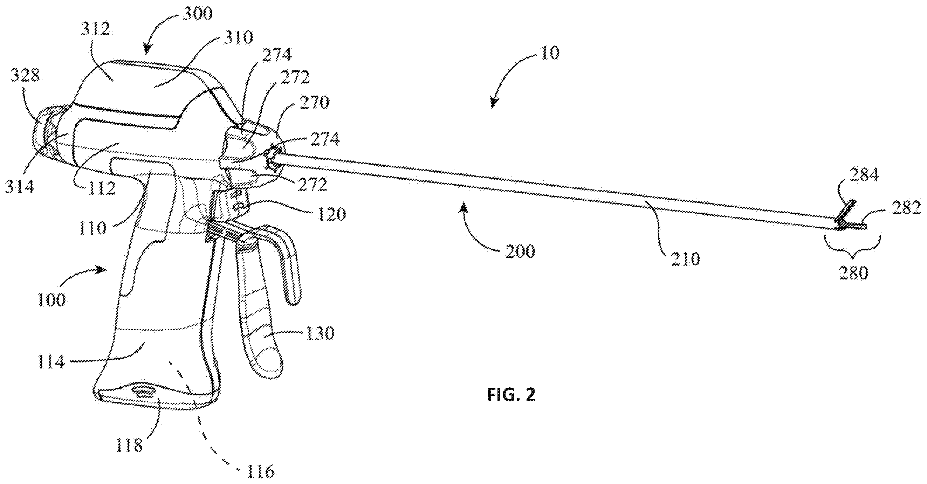

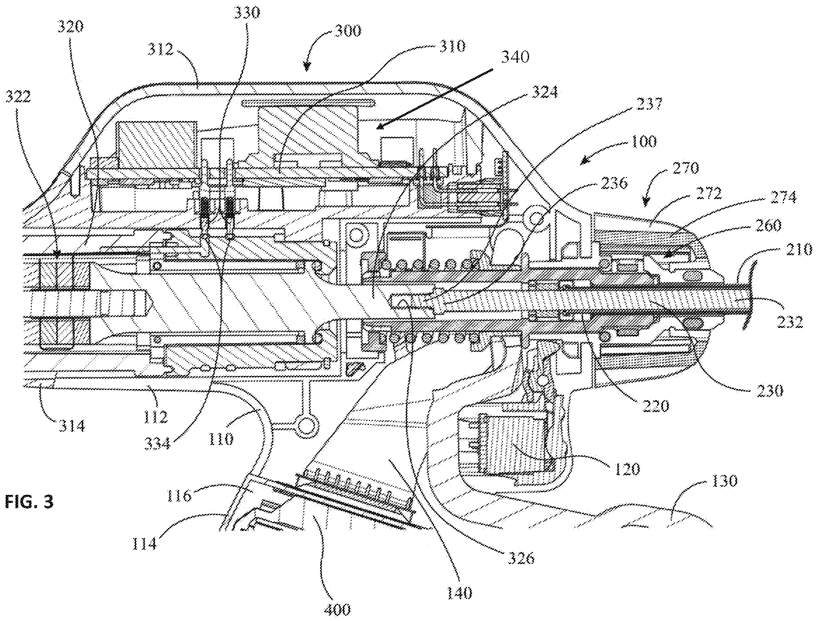

Referring to FIGS. 2 and 3, cordless ultrasonic surgical instrument 10 includes a handle assembly 100 and an elongated assembly 200 extending distally from handle assembly 100. Handle assembly 100 includes a housing 110 defining a body portion 112 and a fixed handle portion 114. Handle assembly 100 further includes an activation button 120 and a clamp trigger 130.

Body portion 112 of housing 110 is configured to releasably support an ultrasonic transducer and generator assembly ("TAG") 300 including a generator 310 and an ultrasonic transducer 320. Generator 310 includes a housing 312 configured to house the internal electronics 340 of generator 310, and a cradle 314 configured to rotatably support ultrasonic transducer 320. Ultrasonic transducer 320 includes a piezoelectric stack 322 and a distally-extending horn 324. Horn 324 defines a threaded female receiver 326. A set of connectors 330 and corresponding rotational contacts 334 associated with generator 310 and ultrasonic transducer 320, respectively, enable drive signals to be communicated from generator 310 to piezoelectric sack 322 of ultrasonic transducer 320 to drive ultrasonic transducer 320. Ultrasonic transducer 320 further includes a rotation knob 328 extending proximally therefrom that, when rotated, rotates ultrasonic transducer 320 relative to generator 310 and housing 110.

Fixed handle portion 114 of housing 110 defines a compartment 116 configured to receive a battery assembly 400 and a door 118 configured to enclose compartment 116. An electrical connection assembly 140, e.g., a flex circuit, is disposed within housing 110 of handle assembly 100 and serves to electrically couple activation button 120, generator 310 of TAG 300, and battery assembly 400 with one another when TAG 300 is supported on or in body portion 112 of housing 110 and battery assembly 400 is disposed within compartment 116 of fixed handle portion 114 of housing 110, thus enabling activation of ultrasonic surgical instrument 10 in response to depression of activation button 120.

Referring still to FIGS. 2 and 3, elongated assembly 200 includes an outer drive sleeve 210, an inner support sleeve 220 disposed within outer drive sleeve 210, a waveguide 230 extending through inner support sleeve 220, a drive assembly 250, an integrated torque assembly 260, a rotation knob 270, and an end effector 280 including a blade 282 and a jaw 284. A proximal portion of outer drive sleeve 210 is operably coupled to clamp trigger 130 of handle assembly 100 via drive assembly 250, while a distal portion of outer drive sleeve 210 is operably coupled to jaw 284. As such, clamp trigger 130 is selectively actuatable to thereby move outer drive sleeve 210 about inner support sleeve 220 to pivot jaw 284 relative to blade 282 of end effector 280 from a spaced-apart position to an approximated position for clamping tissue between jaw 284 and blade 282. Other suitable configurations are also contemplated. Drive assembly 250 provides a force-limiting feature whereby the clamping pressure applied to tissue is limited to a particular clamping pressure or clamping pressure within a particular clamping pressure range. Rotation knob 270 defines a plurality of alternating flutes 272 and protrusions 274 arranged annularly about an exterior surface of rotation knob 270.

Waveguide 230, as noted above, extends through inner support sleeve 220. Waveguide 230 defines a body 232 and blade 282 extending from the distal end of body 232. Blade 282 serves as the blade 282 of end effector 280. Waveguide 230 further includes a proximal connector 236 configured to enable engagement of waveguide 230 with horn 324 of ultrasonic transducer 320 such that ultrasonic motion produced by ultrasonic transducer 320 is transmitted along waveguide 230 to blade 282 for treating tissue clamping between blade 282 and jaw 284 or positioned adjacent to blade 282. To this end, proximal connector 236 includes a threaded male shaft 237 that is configured for threaded engagement within threaded female receiver 326 of horn 324 of ultrasonic transducer 320, although other suitable engagements, releasable or permanent, between horn 324 and waveguide 230 are also contemplated.

In order to facilitate ultrasonic energy transmission, waveguide 230 and ultrasonic transducer 320 are sufficiently engaged with one another without over-tightening. Integrated torque assembly 260 helps ensure that waveguide 230 and ultrasonic transducer 320 are sufficiently engaged while inhibiting over-tightening, although removable torque assemblies are also contemplated. More specifically, integrated torque assembly 260 is operably coupled about outer drive sleeve 210, inner support sleeve 220, and waveguide 230 such that rotation of rotation knob 270 relative to handle assembly 100 rotates elongated assembly 200 relative to handle assembly 100 up to a torque threshold, at which point integrated torque assembly 260 decouples rotation knob 270 from the other components of elongated assembly 200 such that further rotation of rotation knob 270 does not impart rotation to the other components of elongated assembly 200.

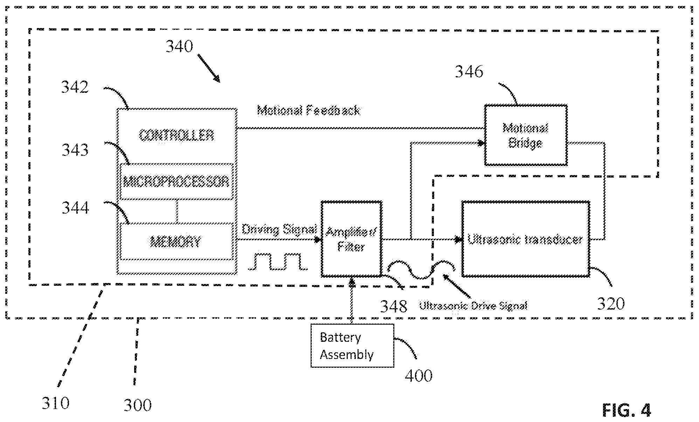

With additional reference to FIG. 4, generator electronics 340 of generator 310 are powered by battery assembly 400 and include a controller 342 configured to control the ultrasonic drive signal output to transducer 320. Controller 342, more specifically, includes a microprocessor 343 and memory 344, e.g., storing instructions to be executed by microprocessor 343 to control the ultrasonic drive signal provided to transducer 320 based on received motional feedback. A motional bridge 346 is configured to sense a mechanical motion, e.g., a magnitude and frequency of mechanical motion, of transducer 320 (although other suitable mechanical motion sensing features are also contemplated). The mechanical motion feedback provided by motional bridge 346 to controller 342 enables controller 342 to control the frequency and/or magnitude of the driving signal provided to an amplifier/filter 348 so that amplifier/filter 348, in turn, provides a suitable drive signal to transducer 320 to drive transducer 320 at its resonance frequency and to achieve a target amount of mechanical motion of ultrasonic transducer 320 at its resonance frequency. Controller 342 is also configured to monitor the resonant frequency of transducer 320, which varies throughout use such as, for example, due to changes in load applied to blade 282 (FIG. 2), temperature of blade 282 (FIG. 2), and/or other factors.

Referring generally to FIGS. 2-4, the assembly, use, and disassembly of cordless ultrasonic surgical instrument 10 are detailed. Initially, TAG 300 is engaged with body portion 112 of housing 110 of handle assembly 100 such that the appropriate contacts associated with TAG 300 are electrically coupled with corresponding contacts of electrical connector 140 of housing 110. Thereafter, or prior to engagement of TAG 300, battery assembly 400 is aseptically transferred, e.g., using a guide, into and engaged within compartment 116 of fixed handle portion 114 of housing 110 of handle assembly 100 such that the appropriate contacts associated with battery assembly 400 are electrically coupled with corresponding contacts of electrical connector 140 of housing 110.

Next, rotation knob 328 of ultrasonic transducer 320 is grasped with one hand, so as to stabilize and inhibit rotation of ultrasonic transducer 320, while rotation knob 270 of elongated assembly 200 is grasped with the other hand. Rotation knob 270 of elongated assembly 200 is then rotated in an engagement direction to threadingly engage waveguide 230 and ultrasonic transducer 320 with one another. Rotation of rotation knob 270 is continued until sufficient engagement between waveguide 230 and ultrasonic transducer 320 is achieved. At this point, further rotation of rotation knob 270 causes integrated torque wrench 260 to slip, thereby inhibiting application of additional torque. An audible and/or tactile "click" may be produced to indicate that the torque threshold has been reached.

Cordless ultrasonic surgical instrument 10, as assembled above, is now ready for use. In use, ultrasonic instrument 10 is inserted into and manipulated within a surgical site such that end effector 280 is positioned adjacent tissue to be treated. If needed, end effector 280 may be rotated relative to handle assembly 100 by rotating rotation knob 270. Once positioned as desired, clamp trigger 130 may be actuated to pivot jaw member 282 from the open position towards the clamping position to clamp tissue to be treated between jaw member 282 and blade 284. As detailed above, drive assembly 250 functions to limit the clamping pressure applied to grasped tissue to a particular clamping pressure or a clamping pressure within a particular clamping pressure range.

With tissue sufficiently clamped between jaw member 282 and blade 284, activation button 120 may be activated in either a first, low power or tissue sealing mode or a second, high power or tissue transection mode to initiate the supply power from battery assembly 400 to TAG 300 for driving ultrasonic transducer 320 to, in turn, produce and transmit ultrasonic mechanical motion along waveguide 230 to blade 284 for treating tissue therewith, e.g., for sealing and/or transecting tissue.

Once the procedure is completed and ultrasonic surgical instrument 10 withdrawn from the surgical site, TAG 300 is disengaged from handle assembly 100 and elongated assembly 200 in the opposite manner as detailed above with respect to the engagement, and is removed from handle assembly 100 for sterilization and/or other cleaning in preparation for further use. Battery assembly 400 is also removed from handle assembly 100 and is cleaned and stored or placed on a charger (not shown) in preparation for further use. Handle assembly 100 and elongated assembly 200 may be discarded or sterilized for subsequent use.

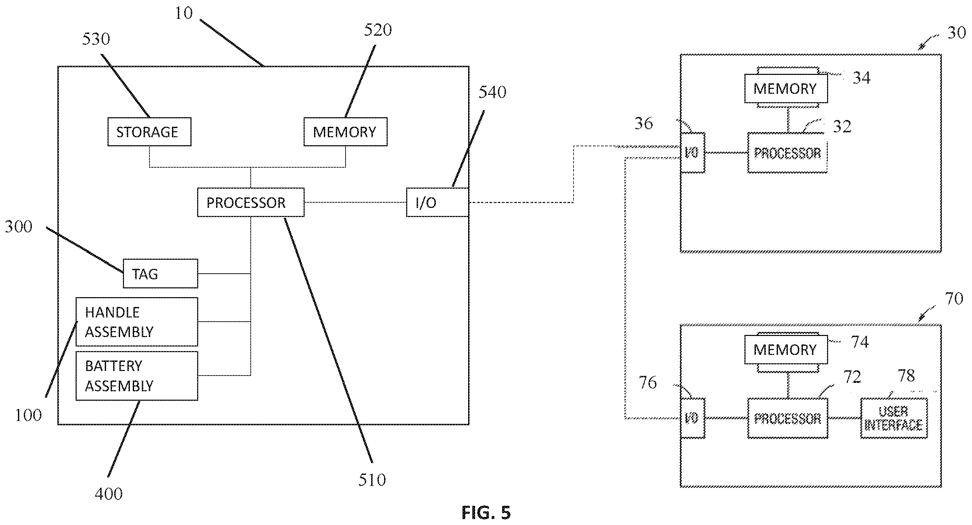

Referring FIG. 5, in conjunction with FIGS. 2 and 3, cordless ultrasonic surgical instrument 10 further includes a processor 510, a memory 520 storing instructions to be executed by processor 510, a storage device 530 storing information relating to cordless ultrasonic surgical instrument 10. Processor 510 and memory 520 may be part of generator electronics 340 (separate from or the same as microprocessor 343 and memory 344, respectively (see FIG. 4)), may be part of battery assembly 400, may be part of handle assembly 100, or may be part of any other portion(s) or component(s) of cordless ultrasonic surgical instrument 10. Likewise, storage device 530 may be part of generator electronics 340, may be part of battery assembly 400, may be part of handle assembly 100, or may be part of any other portion(s) or component(s) of cordless ultrasonic surgical instrument 10.

Processor 510 may include one or more similar or different processors which may be any suitable processor(s) (e.g., control circuit(s)) adapted to perform the operations, calculations, and/or set of instructions described in this disclosure including, but not limited to, a hardware processor, a field programmable gate array (FPGA), a digital signal processor (DSP), a central processing unit (CPU), a microprocessor, and combinations thereof. Memory 520 and storage device 530 may each include one or more of volatile, non-volatile, magnetic, optical, or electrical media, such as read-only memory (ROM), random access memory (RAM), electrically-erasable programmable ROM (EEPROM), non-volatile RAM (NVRAM), or flash memory.

Cordless ultrasonic surgical instrument 10 also includes an input/output (I/O) 540 such as, for example, a wireless transceiver, to enable wireless communication between cordless ultrasonic surgical instrument 10 and communication hub 30. Thus, processor 510 may direct the output of information from cordless ultrasonic surgical instrument 10 to communication hub 30 via I/O 540 and/or may receive information from communication hub 30 via I/O 540.

Various portions of cordless ultrasonic surgical instrument 10 may include electronic components configured to provide information to processor 510. For example, handle assembly 100 may include an EEPROM or other suitable read or read/write storage device (such as those noted above) storing information pertaining to handle assembly 100 such as, for example, identifying data (e.g., serial number, model number, manufacture date, etc.) and/or use data (e.g., whether used or number of uses, TAG's used therewith, battery assemblies used therewith, etc.). As another example, battery assembly 400 may include a control chip that controls charging and discharging of the battery cells of battery assembly 400, implements safety protections (for over current, over voltage, temperature limits, etc.), etc. The control chip may include a storage device (such as those noted above) that stores identifying data (e.g., serial number, model number, manufacture date, etc.), use data (e.g., number of uses and/or sterilizations, number of charges, amount of time used, TAG's used therewith, handle assemblies used therewith, etc.) and/or performance data (e.g., charge and discharge information, fault logging, etc.). Further, as still another example, generator electronics 340 may include a storage device (such as those noted above) storing identifying data (e.g., serial number, model number, manufacture date, etc.), use data (e.g., number of uses, amount of time used, battery assemblies used therewith, handle assemblies used therewith, etc.) and/or performance data (e.g., driving signal information, transducer operation information, activation information, generator operating parameters, fault logging, etc.). Alternative or additional electronic components associated with removable and/or integrated portions of cordless ultrasonic surgical instrument 10 are also contemplated. The electronic components, e.g., of handle assembly 100, battery assembly 400, and/or TAG 300, may communicate with one another and/or processor 510 via electrical connector 140 or any other suitable wired or wireless connection. In addition to communicating identifying, use, and/or performance data, present signals may be communicated to processor 510, actively or passively, to enable processor 510 to indicate when the various components are connected to one another and/or other components of cordless ultrasonic surgical instrument 10.

Continuing with reference to FIG. 5, communication hub 30 includes a processor 32 and a memory 34 storing instructions to be executed by processor 32. Any suitable one or more processors 32 and memories 34 may be provided, such as those noted above. Communication hub 30 further includes at least one I/O 36 such as, for example, a wireless transceiver, one or more wired communication ports, etc. The at least one I/O 36 enables communication hub 30 to communicate wirelessly with cordless ultrasonic surgical instrument 10 and to communicate via a wired or wireless connection with the one or more connected devices 40-70 (FIG. 1), e.g., display monitor 70. Display monitor 70 likewise includes a processor 72, a memory 74 storing instructions to be executed by processor 72, and an I/O 76 to enable communication with communication hub 30 and/or other devices. Display monitor 70 further includes a user interface (UI) 78, e.g., a display screen.

In aspects, communication hub 30 is configured to receive information from cordless ultrasonic surgical instrument 10, to process the information, and to direct a corresponding output on one or more of the connected devices 40-70 (FIG. 1), e.g., the display of information on UI 78 of display monitor 70, activating an audible notification, etc., based upon the information received from cordless ultrasonic surgical instrument 10. In aspects, communication hub 30 is further configured to receive information from one or more of the connected devices 40-70 (FIG. 1) to facilitate processing the information received from cordless ultrasonic surgical instrument 10 and/or to facilitate directing the corresponding output. For example, a user may input information into one or more of the connected devices 40-70 (FIG. 1), e.g., in response to a prompt from communication hub 30.

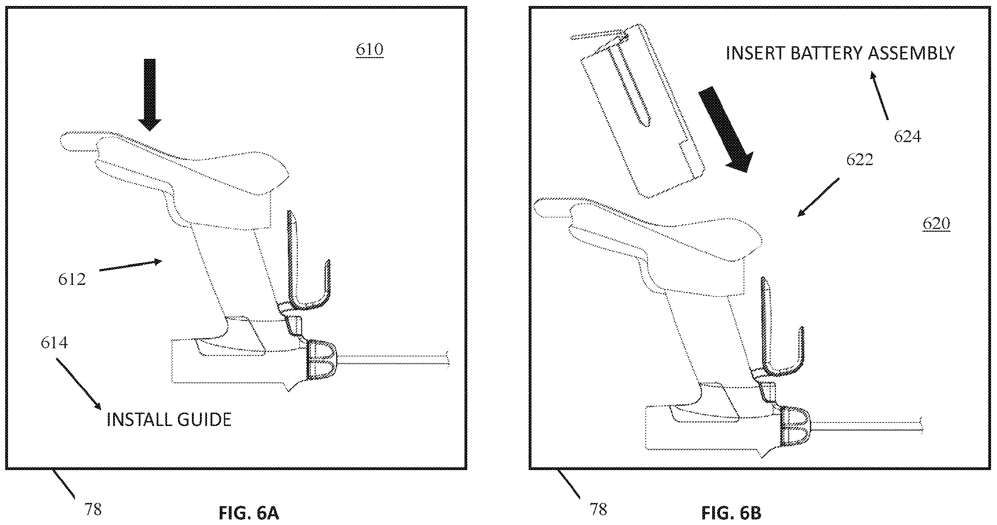

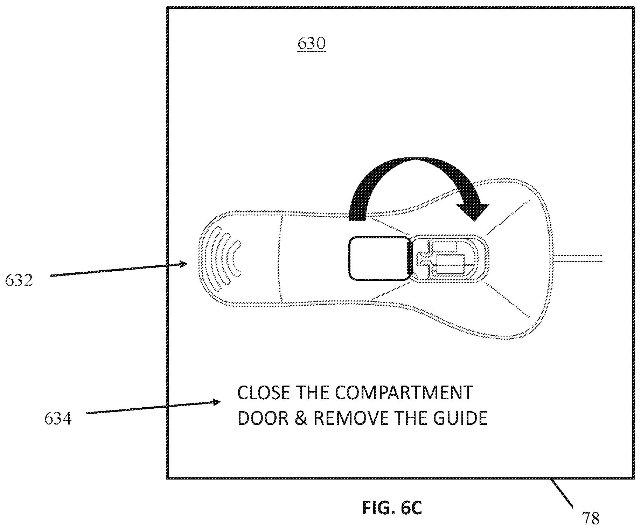

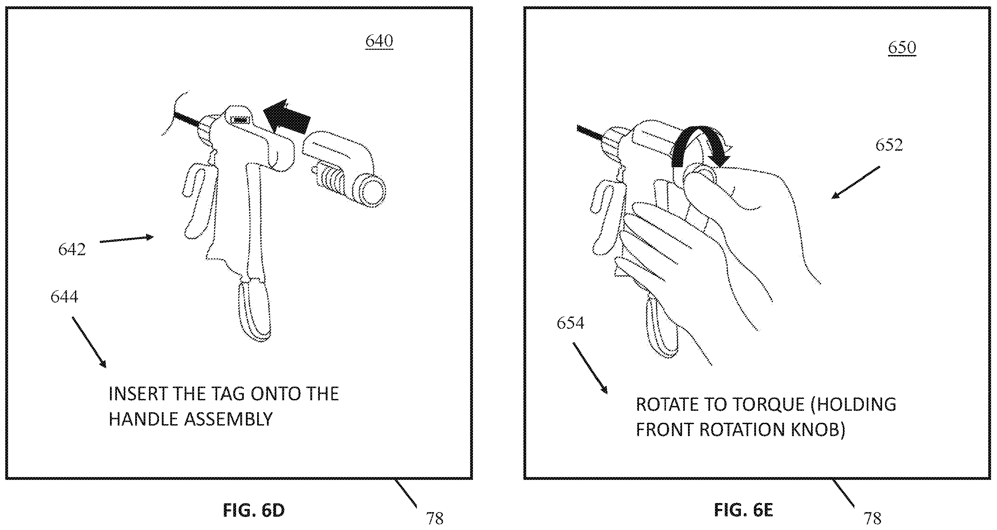

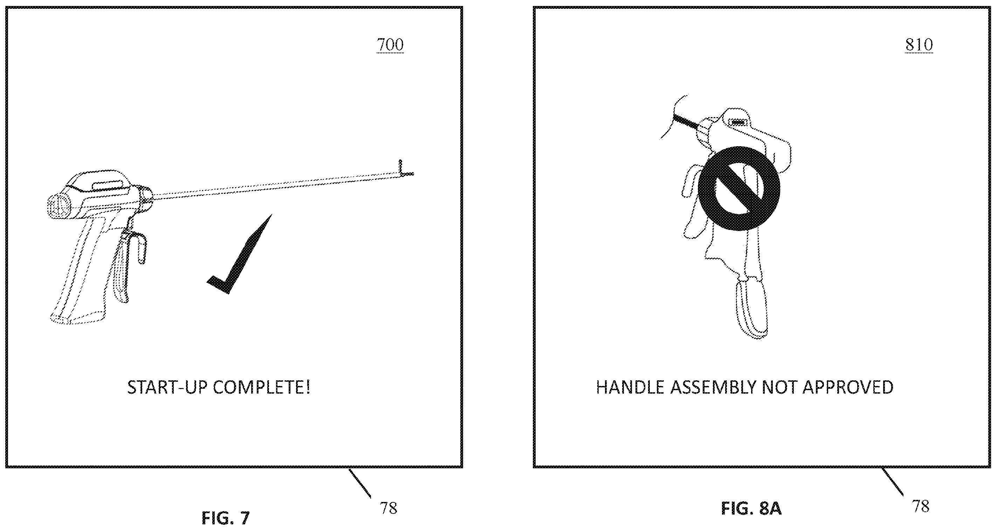

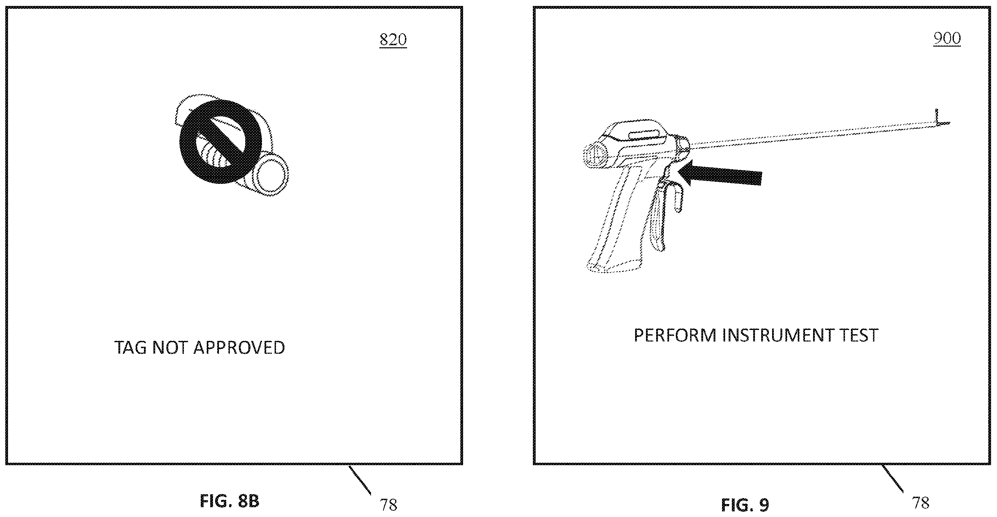

Turning to FIGS. 6-12, in conjunction with FIGS. 1-5, as noted above, communication hub 30 is configured to receive information from cordless ultrasonic surgical instrument 10 and/or one or more of the connected devices 40-70, to process the information, and to direct a corresponding output. For example, as described in greater detail below, communication hub 30 may be configured, based upon the received information, to direct an output, e.g., as a visual display screen on UI 78 of display monitor 70: indicating steps to assemble cordless ultrasonic surgical instrument 10 (see, e.g., FIGS. 6A-6E); indicating completion of a start-up check or detected start-up errors (see, e.g., FIGS. 7-8B); promoting a user to perform an instrument check and indicating the results of the instrument check (see, e.g., FIGS. 9-11); indicating steps to replace battery assembly 400 of cordless ultrasonic surgical instrument 10 (see, e.g., FIGS. 6A-6E); indicate steps to disassemble cordless ultrasonic surgical instrument 10; indicating troubleshooting recommendations; and/or indicating the availability of component, software, firmware, and/or hardware updates.

With reference to FIGS. 6A-6E, in conjunction with FIGS. 1-5, in preparation for performing a surgical procedure, communication hub 30 may direct display monitor 70 to display assembly steps for cordless ultrasonic surgical instrument 10 on UI 78. UI 78, more specifically, may display screens 610, 620, 630, 640, 650 including graphics 612, 622, 632, 642, 652 and/or text 614, 624, 634, 644, 654, respectively, indicating the sequential steps of assembly for cordless ultrasonic surgical instrument 10. An initial step or steps may be displayed upon receipt, at communication hub 30, of a user input, e.g., from one or more of the connected devices 40-70, indicating that cordless ultrasonic surgical instrument 10 is to be assembled. The initial steps may include, for example one or more of: positioning the guide about the instrument, as indicated on screen 610 (FIG. 6A); inserting the battery through the guide into the compartment of the instrument, as indicated on screen 620 (FIG. 6B); closing the door to retain the battery within the compartment of the instrument, as indicated on screen 630 (FIG. 6C); and sliding the TAG onto the handle assembly, as indicated on screen 640 (FIG. 6D).

The initial step or steps may be sequentially displayed on UI 78 with each screen 610, 620, 630, and/or 640 being displayed for a pre-determined amount of time (on a continuous loop and/or including sub-loops). Alternatively, user input, e.g., from one or more of the connected devices 40-70, may be required to move from one screen 610, 620, 630, and/or 640 to the next screen 610, 620, 630, and/or 640 in sequence (and/or to go back to previous screens 610, 620, 630, and/or 640).

As another alternative, information received at communication hub 30 from cordless ultrasonic surgical instrument 10 (or portions thereof) may be utilized to determine when to move from one screen 610, 620, 630, and/or 640 to the next screen 610, 620, 630, and/or 640 in sequence and/or from one group of screens 610, 620, 630, and/or 640 to the next group of screens 610, 620, 630, and/or 640. For example, screens 610-630 may initially be displayed (for a pre-determined amount of time, on a loop, in response to user input, etc.). Once the steps associated with screens 610-630 are completed and, thus, battery assembly 400 is engaged within handle assembly 100, I/O 540 (in aspects where electronic components 510-540 are part of battery assembly 400 or handle assembly 100) may communicate a signal to communication hub 30 indicating that battery assembly 400 and handle assembly 100 are connected to one another. Upon receiving this signal, communication hub 30 provides instructions to move to screen 640, for example.

Screen 640 directs the user to assemble TAG 300 to the handle assembly 100. Once TAG 300 is engaged with handle assembly 100, I/O 540 may communicate a signal to communication hub 30 indicating that handle assembly 100, TAG 300, and battery assembly 400 are connected to one another. Upon receiving this signal, communication hub 30 provides instructions to move to screen 650, for example. Screen 650 illustrates to the user how to torque transducer 320 of TAG 300 to engage transducer 320 and waveguide 230 with one another. It is noted that the above-detailed assembly and corresponding screens 610-650 are exemplary and that additional or alternative assembly steps and/or corresponding screens may be provided.

Referring to FIGS. 7-8B, in conjunction with FIGS. 1-5, once communication hub 30 determines that assembly is complete, e.g., based on receipt of a signal from I/O 540 of cordless ultrasonic surgical instrument 10 indicating the same, communication hub 30 may request information from cordless ultrasonic surgical instrument 10 regarding, for example, handle assembly 100, TAG 300, and battery assembly 400 to perform a start-up check, e.g., to determine that handle assembly 100, TAG 300, and battery assembly 400 are approved for use and/or compatible for use with one another. Such information may include identification information (to determine whether the components are on a blocked list and/or to determine whether the components are capable of being used with one another), use information (to determine whether the components are permitted to be used further), and/or performance information (to determine whether there are any faults with the components). Alternatively, cordless ultrasonic surgical instrument 10 may perform the start-up check and communicate the results to communication hub 30.

Where cordless ultrasonic surgical instrument 10 passes the start-up check, communication hub 30 may provide instructions to output display screen 700 on UI 78. Alternatively, if cordless ultrasonic surgical instrument 10 did not pass the start-up check, communication hub 30 may provide instructions to output a display screen on UI 78 that notifies the user as to the error(s). For example, communication hub 30 may provide instructions to output display screen 810 on UI 78 where the handle assembly 100 is not approved for use (e.g., as a result of being previously used), or provide instructions to output display screen 820 on UI 78 where TAG 300 is not approved for use (e.g., as a result of failure to authenticate generator 320, as a result of an error condition, etc.). Further, where applicable, UI 78 may include a notification indicating the availability of component, software, firmware, and/or hardware updates, regardless of whether cordless ultrasonic surgical instrument 10 passed the start-up check. Additionally, or alternatively, troubleshooting recommendations may be provided if cordless ultrasonic surgical instrument 10 does not pass the start-up check (or any other errors are detected during the start-up check, instrument check, or at any other point).

Turning to FIG. 9, in conjunction with FIGS. 1-5, if cordless ultrasonic surgical instrument 10 passes the start-up check, or in aspects where the start-up check is not provided, communication hub 30 may provide instructions to output display screen 900 on UI 78, to direct the user to perform an instrument test or to indicate that an instrument test will be performed. Communication hub 30 may, in aspects, communicate with cordless ultrasonic surgical instrument 10 to perform the instrument test automatically or upon user-initiation, e.g., depression of activation button 120. The instrument test may include, for example, a brief controlled activation of cordless ultrasonic surgical instrument 10 to determine whether cordless ultrasonic surgical instrument 10 is operating properly, e.g., that resonance can be achieved within a pre-determined time upon activation and/or maintained, that the motional feedback indicates an appropriate output based on the drive signal provided, that power, current, and voltages associated with battery assembly 400 and/or TAG 300 are within acceptable ranges, etc. Cordless ultrasonic surgical instrument 10 may provide the information to communication hub 30 for the instrument test as pass/fail information or real-time data associated with the activation of cordless ultrasonic surgical instrument 10, e.g., resonance, power, voltage, etc. Such real-time data (as well as such pass/fail data) may additionally or alternatively be communicated from cordless ultrasonic surgical instrument 10 to communication hub 30 at other times not limited to the instrument test, such as during use in surgery.

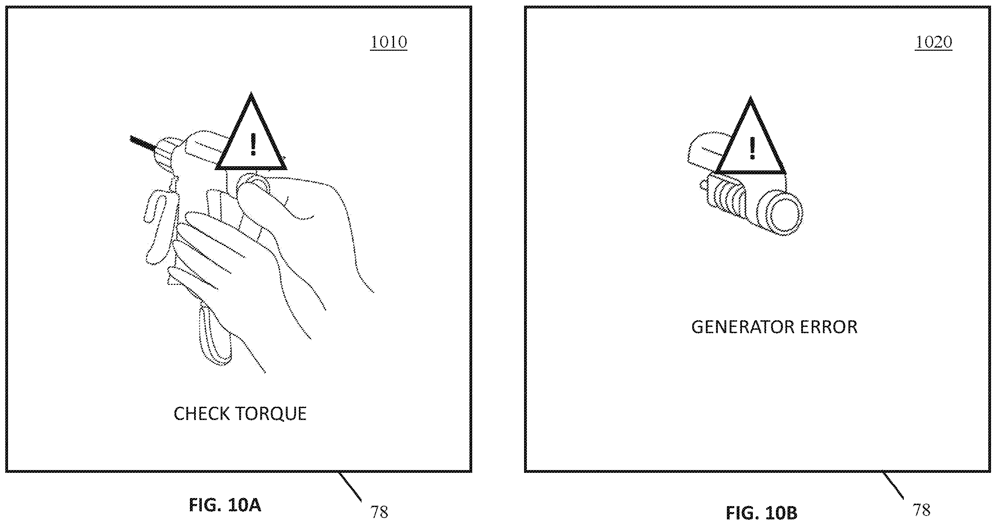

With reference to FIGS. 10A-11, in conjunction with FIGS. 1-5, if cordless ultrasonic surgical instrument 10 fails the instrument test, communication hub 30 may provide instructions to output a display screen on UI 78 that notifies the user as to the error(s). For example, where a potentially under torque condition is detected, display screen 1010 may be provided. As another example, where an error with generator 310 or transducer 320 is detected, display screen 1020 may be provided. If cordless ultrasonic surgical instrument 10 passes the instrument test, display screen 1100 may be provided.

Before, during, or after the start-up test and/or the instrument test, or any other suitable point during setup or use, communication hub 30 may retrieve from cordless ultrasonic surgical instrument 10 the current settings associated with cordless ultrasonic surgical instrument 10 and provide instructions to a user input device, e.g., UI 78, to display, output, or otherwise enable a user interface from which the settings may be viewed, set, modified, and/or confirmed. Additionally or alternatively, a request may be input to a user input device associated with communication hub 30, e.g., UI 78, to display, output, or otherwise enable a user interface from which the current settings associated with cordless ultrasonic surgical instrument 10 may be viewed, set, modified, and/or confirmed. The request for viewing, setting, modifying, and/or confirming the settings and/or the viewing, setting, modifying, and/or confirming of the settings may be any suitable interactions with the user input device (e.g., UI 78) such as, as voice commands, gestures, inputs to cordless ultrasonic surgical instrument 10, or any other suitable interactions. In response to any received setting or modification to settings associated with cordless ultrasonic surgical instrument 10, communication hub 30 provides instructions to cordless ultrasonic surgical instrument 10 to adjust the settings accordingly. Thus, wireless (and, in aspects, remote) adjustment of the settings associated with cordless ultrasonic surgical instrument 10 can be viewed, set, modified, and/or confirmed, e.g., using user interfaces that are more easily navigated as compared to the user interface(s) of cordless ultrasonic surgical instrument 10 itself.

The settings associated with cordless ultrasonic surgical instrument 10 that may be viewed, set, modified, and/or confirmed by the user via communication hub 30 may include, without limitation: assigning different energy levels or modes to each of the activated positions of button 120 (FIG.2); disabling or enabling certain energy levels or modes; setting the corresponding energy levels (blade velocities, for example) associated with the different activation levels (min & max, for example); modifying the brightness of any LED indicators; modifying the volume of any audible indicators; selecting notification and/or alarm profiles (or individual settings) to indicate the notifications and/or alarms to be provided and/or the manner of the notifications and/or alarms; etc.

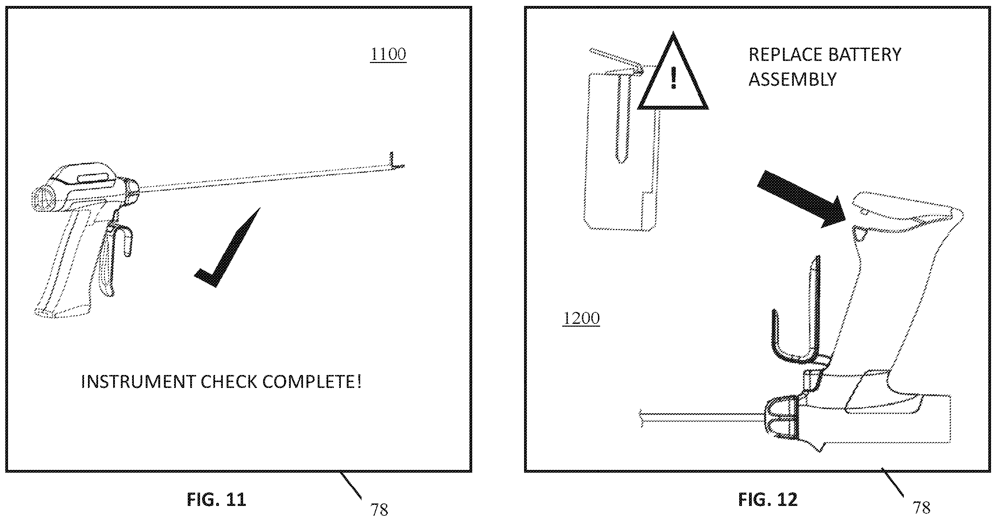

Referring to FIG. 12, in conjunction with FIGS. 1-5, communication hub 30 may provide instructions to output a display screen on UI 78 to notify the user as to one or more conditions of cordless ultrasonic surgical instrument 10 during a surgical procedure. For example, I/O 540 may continuously, periodically, or upon request, output information relating to a state of charge of battery assembly 400 and/or other information relating to the use or performance of battery assembly 400 to communication hub 30. Communication hub 30, in turn, determines whether battery assembly 400 should be replaced, e.g., based upon whether the state of charge of battery assembly 400 is below a threshold value. Alternatively, cordless ultrasonic surgical instrument 10 may determine when battery assembly 400 should be replaced (e.g., based on if the state of charge of battery assembly 400 is below a threshold value) and may output an indication of the same to communication hub 30 via I//O 540. In either configuration, when it is determined that battery assembly 400 should be replaced, communication hub 30 may provide instructions to output one or more display screens, e.g., display screen 1200, on UI 78, notifying the user that battery assembly 400 should be replaced and indicating the steps for removal and/or replacement of battery assembly 400.

Turning back to FIGS. 1-5, after a surgical procedure is completed, communication hub 30 may additionally or alternatively be configured to receive information from cordless ultrasonic surgical instrument 10 and, in response thereto, to direct display monitor 70 to display disassembly steps for cordless ultrasonic surgical instrument 10 in one or more display screens on UI 78. The disassembly steps may be, for example, the opposite of the above-detailed assembly steps (in opposite sequence). Screens associated with disassembly may be displayed for a pre-determined time before moving to the next screen, UI 78 may move to the next screen in response to user input, or UI 78 may move to the next screen in response to information received from cordless ultrasonic surgical instrument 10, similarly as detailed above.

The display screens detailed herein may be static or animated and may include real images, videos, or any other suitable representations of instruments or components. Further, either or any suitable combination of both graphics and text may be provided. The particular display screens and actions or conditions represented thereby that are detailed above are exemplary as it is understood that various modifications may be made to the aspects and features disclosed herein. Therefore, the above description should not be construed as limiting, but merely as exemplifications of various configurations. Those skilled in the art will envision other modifications within the scope of this invention, which is defined by the claims appended hereto.

## Claims

1. A surgical system (2), comprising:
a cordless surgical (10) instrument comprising a processor (510), a memory (520), and an input/output (I/O) (36,540) interface configured for wireless communication, wherein the cordless surgical (10) instrument is configured to obtain information regarding preparation of the cordless surgical instrument for use, the information including information regarding assembly of the cordless surgical instrument, ;
a connected device (40), (50), (60), (70) comprising a processor (72), an input/output (I/O) interface (76), and a user interface (78); and
a communication hub (30) comprising a processor (32) and an input/output (I/O) interface (36), the communication hub (30) configured to wirelessly connect to the cordless surgical instrument to receive the information therefrom, the communication hub configured to instruct, based at least on the information, the connected device configured to provide an output via the user interface (78) relating to the preparation of the cordless surgical instrument for use,
and wherein the output includes display of a plurality of steps for assembling the cordless surgical instrument for use, and wherein the display is configured to switch between different steps of the plurality of steps based on additional information wirelessly communicated from the cordless surgical instrument to the communication hub.

2. The surgical system according to claim 1, wherein the information regarding assembly of the cordless surgical instrument includes information indicating that first and second components of the cordless surgical instrument are engaged with one another.

3. The surgical system according to claim 1, wherein the information regarding preparation of the cordless surgical instrument for use includes identifying information for a plurality of components of the cordless surgical instrument.

4. The surgical system according to claim 3, wherein the output includes display of an indication of whether the plurality of components of the cordless surgical instrument is approved for use.

5. The surgical system according to claim 1, wherein the information regarding preparation of the cordless surgical instrument for use includes information regarding an instrument test of the cordless surgical instrument.

6. The surgical system according to claim 5, wherein the output includes display of an indication of whether the cordless surgical instrument passed or failed the instrument test

7. The surgical system according to claim 6, wherein, in a case where the cordless surgical instrument failed the instrument test, the output further includes at least one of an indication of a cause of the failed instrument test or a troubleshooting recommendation.

8. The surgical system according to claim 1, wherein the cordless surgical instrument includes a battery assembly (400) and a generator (310), the generator configured to drive the cordless surgical instrument, the generator powered by the battery assembly.

9. The surgical system according to claim 8, wherein the cordless surgical instrument further includes an ultrasonic transducer (320) and an ultrasonic blade (282) coupled to the ultrasonic transducer, the generator configured to drive the ultrasonic transducer to produce mechanical vibration motion at the ultrasonic blade.

## Patentansprüche

1. Chirurgisches System (2), umfassend:
ein schnurloses chirurgisches Instrument (10), umfassend einen Prozessor (510), einen Speicher (520) und eine Eingabe/Ausgabe (EA)-Schnittstelle (36, 540), die für drahtlose Kommunikation konfiguriert ist, wobei das schnurlose chirurgische (10) Instrument konfiguriert ist, um Informationen bezüglich der Vorbereitung des schnurlosen chirurgischen Instruments zur Verwendung zu erhalten, wobei die Informationen Informationen bezüglich des Zusammenbaus des schnurlosen chirurgischen Instruments einschließen:
eine verbundene Vorrichtung (40), (50), (60), (70), umfassend einen Prozessor (72), eine Eingabe/Ausgabe (EA)-Schnittstelle (76) und eine Benutzerschnittstelle (78); und
einen Kommunikationshub (30), umfassend einen Prozessor (32) und eine Eingabe/Ausgabe (E/A)-Schnittstelle (36),
wobei der Kommunikationshub (30) konfiguriert ist, um sich drahtlos mit dem schnurlosen chirurgischen Instrument zu verbinden, um dessen Informationen zu empfangen,
wobei der Kommunikationshub konfiguriert ist, um basierend mindestens auf den Informationen die verbundene Vorrichtung anzuweisen, über die Benutzerschnittstelle (78) eine Ausgabe bereitzustellen, die sich auf die Vorbereitung des schnurlosen chirurgischen Instruments zur Verwendung bezieht, und
wobei die Ausgabe die Anzeige einer Vielzahl von Schritten zum Zusammenbauen des schnurlosen chirurgischen Instruments zur Verwendung einschließt, und
wobei die Anzeige konfiguriert ist, um zwischen unterschiedlichen Schritten der Vielzahl von Schritten basierend auf zusätzlichen Informationen, die drahtlos von dem schnurlosen chirurgischen Instrument an den Kommunikationshub übermittelt werden, umzuschalten.

2. Chirurgisches System nach Anspruch 1, wobei die Informationen bezüglich des Zusammenbaus des schnurlosen chirurgischen Instruments Informationen einschließen, die angeben, dass erste und zweite Komponenten des schnurlosen chirurgischen Instruments miteinander in Eingriff stehen.

3. Chirurgisches System nach Anspruch 1, wobei die Informationen bezüglich der Vorbereitung des schnurlosen chirurgischen Instruments zur Verwendung identifizierende Informationen für eine Vielzahl von Komponenten des schnurlosen chirurgischen Instruments einschließen.

4. Chirurgisches System nach Anspruch 3, wobei die Ausgabe die Anzeige einer Angabe einschließt, ob die Vielzahl von Komponenten des schnurlosen chirurgischen Instruments zur Verwendung zugelassen ist.

5. Chirurgisches System nach Anspruch 1, wobei die Informationen bezüglich der Vorbereitung des schnurlosen chirurgischen Instruments zur Verwendung Informationen bezüglich eines Instrumententests des schnurlosen chirurgischen Instruments einschließen.

6. Chirurgisches System nach Anspruch 5, wobei die Ausgabe die Anzeige einer Angabe einschließt, ob das schnurlose chirurgische Instrument den Instrumententest bestanden oder nicht bestanden hat.

7. Chirurgisches System nach Anspruch 6, wobei in einem Fall, in dem das schnurlose chirurgische Instrument den Instrumententest nicht bestanden hat, die Ausgabe ferner mindestens eines von einer Angabe einer Ursache für den nicht bestandenen Instrumententest oder eine Empfehlung zur Fehlerbehebung einschließt.

8. Chirurgisches System nach Anspruch 1, wobei das schnurlose chirurgische Instrument eine Batterieanordnung (400) und einen Generator (310) einschließt, wobei der Generator konfiguriert ist, um das schnurlose chirurgische Instrument anzutreiben, wobei der Generator von der Batterieanordnung mit Strom versorgt wird.

9. Chirurgisches System nach Anspruch 8, wobei das schnurlose chirurgische Instrument ferner einen Ultraschallwandler (320) und eine Ultraschallklinge (282), die an den Ultraschallwandler gekoppelt ist, einschließt, wobei der Generator konfiguriert ist, um den Ultraschallwandler anzutreiben, um an der Ultraschallklinge eine mechanische Vibrationsbewegung zu erzeugen.

## Revendications

1. Système chirurgical (2), comprenant :
un instrument chirurgical sans fil (10) comprenant un processeur (510), une mémoire (520), et une interface d'entrée/sortie (E/S) (36, 540) configurée pour une communication sans fil, dans lequel l'instrument chirurgical (10) sans fil est configuré pour obtenir des informations concernant la préparation de l'instrument chirurgical sans fil en vue d'une utilisation, les informations incluant des informations concernant l'assemblage de l'instrument chirurgical sans fil, ;
un dispositif connecté (40), (50), (60), (70) comprenant un processeur (72), une interface d'entrée/sortie (E/S) (76), et une interface utilisateur (78) ; et
un concentrateur de communication (30) comprenant un processeur (32) et une interface d'entrée/sortie (E/S) (36), le concentrateur de communication (30) étant configuré pour se connecter sans fil à l'instrument chirurgical sans fil pour recevoir les informations de celui-ci, le concentrateur de communication étant configuré pour ordonner, en fonction au moins des informations, au dispositif connecté configuré de fournir une sortie par le biais de l'interface utilisateur (78) relative à la préparation de l'instrument chirurgical sans fil en vue d'une utilisation,
et dans lequel la sortie comporte l'affichage d'une pluralité d'étapes pour assembler l'instrument chirurgical sans fil en vue d'une utilisation, et dans lequel l'affichage est configuré pour basculer entre différentes étapes de la pluralité d'étapes en fonction d'informations supplémentaires communiquées sans fil de l'instrument chirurgical sans fil au concentrateur de communication.

2. Système chirurgical selon la revendication 1, dans lequel les informations concernant l'assemblage de l'instrument chirurgical sans fil comportent des informations indiquant que des premier et second composants de l'instrument chirurgical sans fil viennent en prise l'un avec l'autre.

3. Système chirurgical selon la revendication 1, dans lequel les informations concernant la préparation de l'instrument chirurgical sans fil en vue d'une utilisation comportent des informations d'identification pour une pluralité de composants de l'instrument chirurgical sans fil.

4. Système chirurgical selon la revendication 3, dans lequel la sortie comporte l'affichage d'une indication précisant si la pluralité de composants de l'instrument chirurgical sans fil est approuvée en vue d'une utilisation.

5. Système chirurgical selon la revendication 1, dans lequel les informations concernant la préparation de l'instrument chirurgical sans fil en vue d'une utilisation comportent des informations concernant un test d'instrument de l'instrument chirurgical sans fil.

6. Système chirurgical selon la revendication 5, dans lequel la sortie comporte l'affichage d'une indication précisant si l'instrument chirurgical sans fil a réussi ou échoué au test d'instrument.

7. Système chirurgical selon la revendication 6, dans lequel, dans un cas où l'instrument chirurgical sans fil a échoué au test d'instrument, la sortie comporte en outre au moins l'une parmi une indication d'une cause de l'échec du test d'instrument et une recommandation de dépannage.

8. Système chirurgical selon la revendication 1, dans lequel l'instrument chirurgical sans fil comporte un ensemble batterie (400) et un générateur (310), le générateur étant configuré pour entraîner l'instrument chirurgical sans fil, le générateur étant alimenté par l'ensemble batterie.

9. Système chirurgical selon la revendication 8, dans lequel l'instrument chirurgical sans fil comporte en outre un transducteur à ultrasons (320) et une lame à ultrasons (282) couplée au transducteur à ultrasons, le générateur étant configuré pour attaquer le transducteur à ultrasons pour produire un mouvement de vibration mécanique au niveau de la lame à ultrasons.
